# EUROPEAN PATENT APPLICATION

(11) **EP 2 384 703 A1**
(43) Date of publication of application: **09.11.2011**
(21) Application number: 10382110.4
(22) Date of filing: 05.05.2010
(51) Int. Cl.: A61B 17/00

(54) **Device for dispensing a two-component tissue adhesive or sealant**

(71) Applicant: Bioadhesives Meditech Solutions, S.L., 03206 Elche, Alicante (ES)
(72) Inventor: Martinez Paino, Manuel, 03206, ELCHE (Alicante) (ES); Dominguez Arribas, Luis Esteban, 03206, ELCHE (Alicante) (ES); Martin Martinez, José Miguel, 03206, ELCHE (Alicante) (ES); Cajaraville Zubeldia, Pablo, 03206, ELCHE (Alicante) (ES); Sola Otano, Ignacio Carmelo, 03206, ELCHE (Alicante) (ES)
(74) Representative: ZBM Patents

(57) **Abstract**

Device for dispensing a two-component tissue adhesive or sealant composition, the device comprising a housing, a plunger movable through the housing, a mixing chamber arranged downstream of the housing in a dispensing direction, and at least two cartridges, each suitable for containing one of the components, the cartridges being flexible and adapted to be received in the housing, whereby, upon appropiate movement of the plunger, the cartridges are pressed such that their content is delivered towards the mixing chamber.

## Description

The present invention relates to a device for dispensing a two-component tissue adhesive or sealant, which comprises a housing and a plunger movable through the housing, enabling to apply a mixture of two components forming a tissue adhesive or sealant for a wound, the components being stored separately inside the housing until the product is dispensed.

Several devices for dispensing bio-adhesive mixtures to wounds are known, the mixture being pre-mixed inside the device's housing or being mixed as it is being applied. For example, double-barreled syringes are known, comprising two chambers, and wherein the components of a bio-adhesive mixture are stored inside the chambers to be dispensed forming the bio-adhesive mixture in the outlet of the syringe. However, the syringes' plungers are problematic since the components inside the chambers may react with the rubber found in the surface of the plunger in contact with them, solidifying inside the chamber and difficulting a proper dispensing.

On the other hand, in US20030187387A1 a dispenser is described, comprising two chambers and a moveable plunger, wherein the chambers contain one component each, of a two-component tissue adhesive or sealant. More specifically, each component is stored in a rigid cartridge, which has a plug at one end: in order to apply the adhesive mixture, the plunger is pressed towards the plug of the cartridge, which is displaced and pushes the component inside the cartridge towards a mixing chamber and the outlet of the dispenser.

However, if a good and precise dispensing of the adhesive or sealant is wanted, the plug's cartridges have to properly isolate the inside of the cartridge from the environment at any time, but at the same time, the plug has to be movable along the cartridge. Therefore, the cartridge may be relatively expensive and not simple to manufacture, to meet said requirements.

Also, in US2009/0024104, a syringe is described, comprising two flexible bags arranged aligned inside the syringe housing, and further comprising spikes to pierce the bags. When in use, the two components contained inside the bags are mixed inside the housing and then delivered at one end thereof.

In this case, the mixing performed inside the syringe may be irregular and it may comprise different percentatges of components depending on, for example, how the syringe's plunger is pressed, thus not being reliable for a precise mixing of the components. Furthermore, the resulting product of the mixing of the two components may solidify before reaching the outlet of the syringe, in an intermediate zone of the housing.

### Summary of the invention

It is an object of the present invention to provide a device which at least party alleviates the above problems of the prior art.

According to a first aspect, the present invention relates to a device for dispensing a two-component tissue adhesive or sealant composition, the device comprising a housing, a plunger movable through the housing, a mixing chamber arranged downstream of the housing in a dispensing direction, and at least two cartridges, each suitable for containing one of the components, the cartridges being flexible and adapted to be received in the housing, whereby, upon appropiate movement of the plunger, the cartridges are pressed such that their content is delivered towards the mixing chamber.

The flexible cartridges may be easier and relatively cheap to manufacture and may mantain the products perfectly isolated from the environment until they are finally dispensed. Furthermore, the presence of a mixing chamber downstream the housing with the cartridges allows to deliver the components from the cartridges to the mixing chamber simultaneously and at a regular pace, just before their dispensing from an outlet.

According to the present description, when using the term "component", it is used to refer to a product used for making a mixture, said mixture being a tissue-adhesive or sealant, wherein said product may be formed in turn by a single product or a compound of two or more products. A case may be found, for example, when the tissue-adhesive or sealant is obtained by mixing a component made of two products, and a component made of a single product.

According to an embodiment, at least one of the cartridges comprises a tubular body having a side surface at least partly shaped as a bellows, and therefore, depending on the size and form of the pleats of the bellow, the inner chamber of the bellow may comprise more or less component, enabling to use the same housing with different cartridges depending on the percentage of both components that may have to be found in the bio-adhesive mixture. Also, with this feature, when manufacturing the device, the same housing may be used and only the bellows may be manufactured different depending on the desired final adhesive, saving money on the device's overall manufacturing cost.

According to an embodiment of the invention, the device further comprises at least two chambers each adapted to receive a cartridge.

This way, there is a security that, when each cartridge is opened, there would be no contact between a possible leackage of a component and the other one, mixing the components outside the mixing chamber (that is, in case that the cartridges would be in the same chamber of the housing), provoking the adhesive to be formed inside the housing and difficulting the normal usage of the device.

According to a further embodiment, the mixing chamber is in fluid communication with the chambers. Also, the housing may comprise a plunger for each chamber.

According to an specific embodiment, both plungers form an integral piece of the device, that is, they are joint in the opposite end to the part of the plungers which applies pressure to the cartridges.

According to a further embodiment of the invention, the device comprises at least one opener spike for each cartridge

Also, the device may have three distinct positions: a first one wherein the cartridge is closed and inside its' correspondent chamber, a second one wherein pression is applied to the cartridge by means of the plunger and the end of the cartridge near the mixing chamber is being opened by an opening spike, and a third one wherein the cartridge is fixed to the end its' chamber near the mixing chamber, after a click of the cartridge, and completely opened, so that the user can apply more pressure to make flow the components towards the mixing chamber.

According to another embodiment, the device comprises a mixing element, and the device may be such that both opener spikes and the mixing element form an integral piece, which is arranged in part in the mixing chamber.

Therefore, when pressure is applyied to one end of the flexible cartridge by means of the plunger, the other end of the cartridge, positioned near the mixing chamber and the outlet of the chamber, where the opener spike is located, will be opened.

More specifically, according to a further embodiment, the cartridges may further comprise a stopper element, which may also be provided with a piercebable film.

This way, the stopper tabs are perforated by the opener spikes allowing both components to flow from each cartridge, through an exit conduit, towards the mixing chamber where the adhesive is to be obtained.

According to another embodiment, the interior surface of the housing comprises at least one zone with a rack comprising at least two teeth, and the plunger comprises a pawl adapted to fit the depression formed by the teeth of the rack.

Said rack and pawl are adapted to function in a similar way as a ratchet consisting of a round gear or linear rack with teeth and a pawl that engages the teeth. The teeth may be uniform but asymmetrical, with each tooth having a moderate slope on one edge and a much steeper slope on the other edge.

Therefore, when in use, the teeth are moving in the unrestricted (i.e., forward) direction, and the pawl easily slides up and over the gently sloped edges of the teeth, with a spring forcing it into the depression between the teeth as it passes the tip of each tooth. When the teeth move in the opposite (backward) direction, however, the pawl will catch against the steeply sloped edge of the first tooth it encounters, thereby locking it against the tooth and preventing any further motion in that direction.

Therefore, the unrestricted direction in which the plunger moves is the direction where it performs pressure in the cartridges' surfaces, to make the mixing of the components found within the cartridges, in the mixing chamber, and finally applying the bio-adhesive mixture.

With this, a more precise and slow application of the bio-adhesive mixture may be performed if the user desires so, depending on the size of both the racks and the pawls of the device and the pressure applied to the plunger by the user.

Furthermore, both cartridges may have different diameters, depending on the quantities of the different components required for each bio-adhesive mixture, and also, the chambers may also have different diameters, since it may be usual that one of the cartridges is bigger than the other one.

According to another aspect of the invention, a flexible cartridge for a device as previously described is provided, and, furthermore, according to a further embodiment, the cartridge comprises a tubular body having a side surface at least partly shaped as a bellows.

### Brief description of the Drawings

Particular embodiments of the present invention will be described in the following, only by way of non-limiting example, with reference to the appended drawings, in which:
Figure 1 is an exploded view of a first embodiment of the device for applying a bio-adhesive mixture, according to the present invention;
Figure 2 is a cross-section view of an embodiment of the device for applying a bio-adhesive mixture, according to the invention;
Figure 3 is a cross-section view of an embodiment of the device for applying a bio-adhesive mixture, according to the invention;
Figure 4 is another cross-section of a further embodiment of the device for applying a bio-adhesive mixture, according to the invention;

### Description of preferred embodiments of the invention

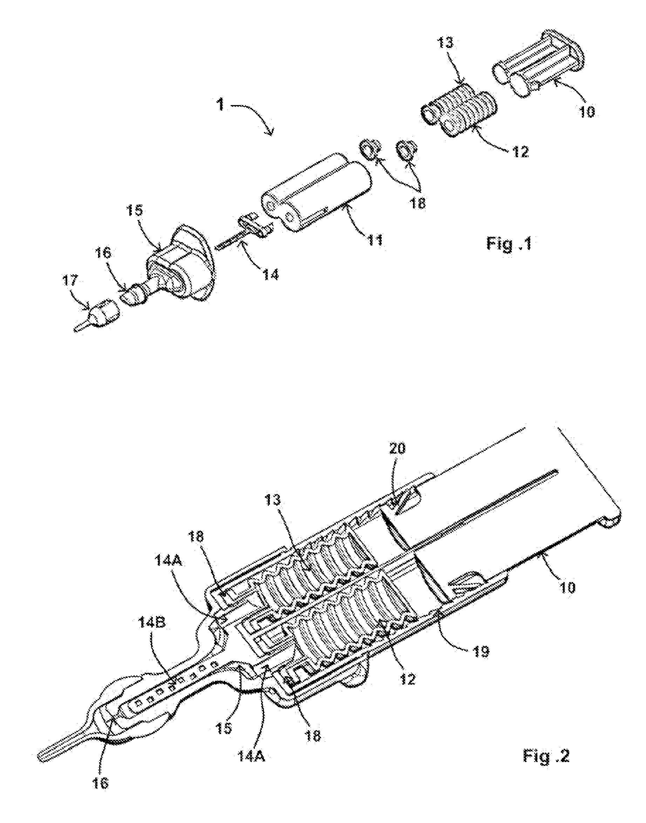

Figure 1 shows a first embodiment according to the present invention. A device for dispensing a tissue adhesive, in the form of a syringe, is indicated with reference sign 1, the device comprising a housing 11 with two chambers adapted to recieve two flexible cartridges 12 and 13, both said cartridges having a bellow type structure and being sealed by a stopper tab 18. Furthermore, the syringe comprises a double-plunger 10 which is embodied in the same piece, and which is positioned in such a way that is suitable for applying pressure in the surface of the bellows which is located opposite to the bellows' surface where the stopper 18 is located. Furthermore, the device comprises an opener spike for each chamber, adapted to perforate each tab of the bellows, and a mixing element 14 which is comprised in the same piece as both opener spikes; a mixing chamber 15 located in the oposite part of the plunger, and which is in fluid connection with the chambers, and an outlet nozzle 16 of the mixing chamber 15, suitable for dispensing the mixture directly to, for example, a patient's skin. Also, this specific embodiment comprises a removable dispensing tip 17 which can be coupled to the nozzle 16 for performing the dispensing of the mixture with a different and narrower dispensing surface.

Figure 2 shows the same embodiment of the device 1 in a cross-sectional view, wherein the bellows 12 and 13 have different sizes. The stopper tabs 18 are already perforated by the opener spikes 14A, which have tube form, in such a way that, when the tab is perforated, the component found inside both bellows 12 and 13, can pass through the tubes formed by the spikes towards the mixing chamber 15, mix together by means of the mixing element 14B, and finally exit through the outlet nozzle of the device 16 and the removable tip 17.

Furthermore, the mixing element 14B comprises an elongated piece with a plurality of holes which make the mixing of the two components easier when they pass through the mixing chamber, until the mixture is done and reaches the nozzle 16, to be dispensed.

Also, the interior of the housing has a rack and pawl system, which consists of a serrated surface 19 partially extended through the interior of the housing, which acts as a rack, and a pawl 20 found in the end of the plunger 10 which applies pressure to the bellows 12 and 13. This way, the rack and pawl system enables the user to press the bellows in small amounts and always in the same direction, making the dispensing of both components to the mixing chamber and the outlet more precise, since the quantities needed for making the mixture of the two components are within the range of several mililiters.

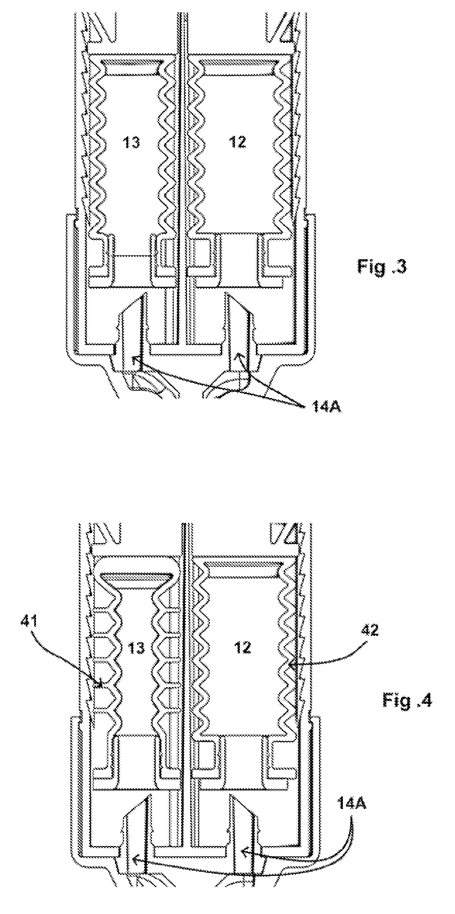

Figure 3 and 4 show two different embodiments of the device according to the present invention, wherein both bellows 12 and 13 are still not opened by the opener spikes 14A, and wherein, in the device in Figure 3, both cartridges have the same structure of their bellows' pleats, and a different bellow diamater, but in Figure 4, the pleats 41 of the first cartridge 13 are more elongated than the pleats 42 of the second cartridge 12 and, in consecuence, the inner chamber of the bellow 13 is smaller than the analog one in the embodiment shown in Figure 3. This way, only by changing the size of the pleats, it is possible to obtain different percentages of components for different bio-adhesive mixtures, and still use and manufacture the same housing and chamber size of the device.

## Claims

1. Device for dispensing a two-component tissue adhesive or sealant composition (1), the device comprising a housing (11), a plunger (10) movable through the housing (11), a mixing chamber (15) arranged downstream of the housing in a dispensing direction, and at least two cartridges (12) (13), each suitable for containing one of the components, the cartridges being flexible and adapted to be received in the housing, whereby, upon appropiate movement of the plunger, the cartridges are pressed such that their content is delivered towards the mixing chamber.

2. Device according to claim 1, wherein at least one of the cartridges comprises a tubular body having a side surface at least partly shaped as a bellows.

3. Device according to claim 1 or 2, **characterized in that** it further comprises at least two chambers each adapted to receive a cartridge.

4. Device according to claim 3, wherein the mixing chamber is in fluid communication with the chambers.

5. Device according to any of claims 3 to 4, wherein the housing comprises a plunger for each chamber.

6. Device according to claim 5, wherein both plungers form an integral piece of the device.

7. Device according to any of claims 1 to 6, further comprising at least one opener spike (14A) for each cartridge.

8. Device according to any of claims 1 to 7, further comprising a mixing element (14B).

9. Device according to claims 7 and 8, wherein both opener spikes (14A) and the mixing element (14B) form an integral piece, which is arranged in part in the mixing chamber (15).

10. Device according to any of claims 1 to 9, wherein the cartridges (12) (13) further comprise a stopper element (18).

11. Device according to claim 10, wherein the stopper element (18) is provided with a piercebable film.

12. Device according to any of claims 1 to 11, wherein the interior surface of the housing comprises at least one zone with a rack comprising at least two teeth (19), and the plunger (10) comprises a pawl (20) adapted to fit the depression formed by the teeth of the rack.

13. Device according to any of claims 3 to 9, wherein both chambers have different diameters.

14. Device according to any of claims 1 to 13, wherein both cartridges (12) (13) have different diameters.

15. A flexible cartridge (12) (13) for a device as claimed in any of claims 1 to 14.

16. A flexible cartridge according to claim 15, further comprising a tubular body having a side surface at least partly shaped as a bellows.
